# EUROPÄISCHE PATENTSCHRIFT

(11) **EP 1 732 499 B1**
(45) Veröffentlichungstag und Bekanntmachung des Hinweises auf die Patenterteilung: **05.03.2014**
(21) Anmeldenummer: 05738066.9
(22) Anmeldetag: 06.04.2005
(51) Int. Cl.: A61K 6/00, A61K 6/083

(54) **HÄRTBARE DENTALMATERIALIEN MIT EINER EINSTELLBAREN TRANSLUZENZ**
HARDENING DENTAL MATERIALS FEATURING ADJUSTABLE TRANSLUCENCE
MATERIAUX DENTAIRES DURCISSABLES A TRANSLUCIDITE AJUSTABLE

(30) Priorität: 07.04.2004 DE 102004017124
(43) Veröffentlichungstag der Anmeldung: 20.12.2006
(73) Patentinhaber: Ivoclar Vivadent AG, 9494 Schaan (LI)
(72) Erfinder: KLAPDOHR, Simone, A-6830 Rankweil (AT); MOSZNER, Norbert, FL-9495 Triesen (DE); VOGEL, Karin, FL-9487 Gamprin-Bendem (LI); BURTSCHER, Peter, A-6830 Rankweil (AT); RHEINBERGER, Volker, FL-9490 Vaduz (LI)
(74) Vertreter: Fitzner, Uwe
(86) Internationale Anmeldenummer: PCT/EP2005/051594
(87) Internationale Veröffentlichungsnummer: WO 2005/097043

(56) Entgegenhaltungen:
- EP-A- 0 732 099
- US-A- 5 936 006
- US-B1- 6 232 367
- US-B1- 6 262 142
- TAIRA M ET AL: "REFRACTIVE INDEX OF INORGANIC FILLERS IN SEVEN VISIBLE-LIGHT-CURED DENTAL COMPOSITE RESINS" JOURNAL OF MATERIALS SCIENCE LETTERS, CHAPMAN AND HALL LTD. LONDON, GB, Bd. 13, 1994, Seiten 68-69, XP001052736 ISSN: 0261-8028
- DATABASE WPI Section Ch, Week 198721 Derwent Publications Ltd., London, GB; Class A12, AN 1987-147642 XP002336599 & JP 62 086003 A (TOKUYAMA SODA KK) 20. April 1987 (1987-04-20)
- DATABASE WPI Section Ch, Week 198839 Derwent Publications Ltd., London, GB; Class A12, AN 1988-273972 XP002336600 & JP 63 199204 A (TOKUYAMA SODA KK) 17. August 1988 (1988-08-17)

## Beschreibung

Die Erfindung betrifft härtbare Dentalmaterialien mit einer einstellbaren Transluzenz und hohen Opaleszenz, insbesondere hochästhetische Füllungsmaterialien.

Aus dem Stand der Technik sind viele Dentalmaterialien bekannt, denen Eigenschaften zugeschrieben werden, die diese als ästhetische Materialien qualifizieren sollen. In der Literatur findet man über solche Eigenschaften, insbesondere über Opaleszenz oder Transluzenz und deren gezielte Einstellung und Variation kaum Informationen.

Ziel von dentalen Restaurationen ist es, die Restauration wie einen natürlichen Zahn aussehen zu lassen. Der natürliche Zahn zeigt verschiedene Transluzenzen, Fluoreszenz und Opaleszenz. Dadurch wirkt der Zahn lebendig. Es gibt permanent das Bemühen, nicht nur die Zahnfarbe und den Farbverlauf in den Restaurationen zu imitieren, mit mehr Farben und verschiedensten Restaurationstechniken zu verbessern, sondern auch fluoreszierende Effekte in das Restaurationsmaterial zu integrieren. Dies wurde in den 90er Jahren auch auf verschiedene Weise versucht, indem z.B. blaue Farbstoffe oder auch mikrofeines-Titandioxid (z.B. EP 533 434) dem herkömmlichen Material zugesetzt wurden.

Die natürliche Farbe des Zahnes wird durch das Dentin bestimmt. Der Opaleszenz-Effekt im Zahn entsteht durch die fast transparente Schmelzschicht. Durch ihre kristalline Struktur bricht sie das Licht derart, dass Opaleszenz auftritt.

Die EP-A-0 732 099 beschreibt Dentalmaterialien mit verbesserter Transparenz. Bei diesem Stand der Technik wird lediglich die Transparenz durch weitgehende Übereinstimmung der Brechungsindizes von Monomermischung und Füllstoffen verbessert. Die Plaquebildung und -anlagerung auf der Zahnoberfläche zu verhindern, ist dieser Entgegenhaltung nicht zu entnehmen.

Aus der US 6,232,367 (Kobashigawa et al.) sind opaleszente Füllstoffe für Dentalmaterialien bekannt, die sich aus einer Mischung eines gemahlenen (üblichen und seit langem bekannten) transluzenten Glasfüller und einem kolloidalen Füllstoff zusammensetzen. Dabei sind die Brechungsindices der Füllstoffe und der verwendeten polymerisierbaren Monomere so aufeinander abgestimmt, dass ein transluzentes Material herstellbar ist. Der Brechungsindex der eingesetzten Monomermischung liegt zwischen 1,45 und 1,60. Die Differenz der Brechungsindices zwischen Monomermischung und Füllstoff beträgt +/- 0,04.

Darüber hinaus ist es aus EP 0 732 099 A bekannt, eine Mischung aus kugelförmigen Partikeln mit asphärischen, splitterförmigen Partikeln zu kombinieren, um bestimmte technische Effekte zu erzielen.

Ziel der Erfindung ist die Herstellung eines opaleszenten Dentalmaterials, bei welchem die erreichbaren Transparenzen und Opaleszenzen im Material gezielt einstellbar sind.

Die erfindungsgemäße Aufgabe wird gelöst durch ein härtbares Dentalmaterial mit einstellbarer Transluzenz und hoher Opaleszenz enthaltend,
I. wenigstens ein radikalisch polymerisierbares fluorsubstituiertes Monomer oder eine Monomermischung enthaltend ein radikalisch polymerisierbares fluorsubstituiertes Monomer mit einem Brechungsindex von <1,45 gemessen bei 25°C,
II. wenigstens einen sphärischen, monodispersen, opaleszenten, anorganischen Füllstoff mit einem Brechungsindex von 1,40 bis 1,45 und einer mittleren Partikelgröße bestimmt mittels Rasterelektronenmikroskopie von 230 nm +/- 50 nm,
III. weitere übliche Füllstoffe oder Füllstoffgemische und
IV. Polymerisationsinitiatoren, Stabilisatoren, Farbstoffe oder Gemische eines oder mehrere dieser Substanzen, wobei
die Füller in Komponente III amorphe kugelförmige Materialien auf Basis von Oxiden sind, wobei
die Monomere den Hauptbestandteil der Dentalmaterialien bilden und die Dentalmaterialien einen Initiator für die radikalische Polymerisation enthalten.

Vorzugsweise enthalten die erfindungsgemäßen härtbaren Dentalmaterialien
I 10 bis 85 Gew.-% polymerisierbares Monomer oder polymerisierbare Monomermischung,
II 10 bis 70 Gew.-% opaleszenten Füllstoff,
III 5 bis 60 Gew.-% weitere übliche Füllstoffe oder Füllstoffgemische und
IV 0,01 bis 5 Gew.-% Polymerisationsinitiatoren, Stabilisatoren, Farbstoffe oder Gemische eines oder mehrerer dieser Substanzen.

Die Dentalmaterialien eignen sich besonders als Zemente, Verblendmaterialien und insbesondere Füllungskomposite.

Ein besonders bevorzugter Zement enthält:
I 10 bis 85 Gew.-%, insbesondere 20 bis 50 Gew.-% polymerisierbares Monomer oder polymerisierbare Monomermischung,
II 10 bis 70 Gew.-%, insbesondere 10 bis 40 Gew.-% opaleszenten Füllstoff,
III 5 bis 50 Gew.-%,-insbesondere 10 bis 40 Ges.-% weitere übliche Füllstoffe oder Füllstoffgemische und
IV 0.01 bis 5 Gew.-%, insbesondere 0.1 bis 2.0 Gew.-% Polymerisationsinitiatoren, Stabilisatoren, Farbstoffe oder Gemische eines oder mehrerer dieser Komponenten,
jeweils bezogen auf die Gesamtmasse des Zements.

Ein besonders bevorzugtes Verblendmaterial enthält:
I 10 bis 50 Gew.-%, insbesondere 10 bis 30 Gew.-% polymerisierbares Monomer oder polymerisierbare Monomermischung,
II 30 bis 70 Gew.-%, insbesondere 40 bis 70 Gew.-% opaleszenten Füllstoff,
III 20 bis 50 Gew.-%, insbesondere 30 bis 40 Gew.-% weitere übliche Füllstoffe oder Füllstoffgemische und
IV 0,01 bis 5 Gew.-%, insbesondere 0,1 bis 2,0 Gew.-% Polymerisationsinitiatoren, Stabilisatoren, Farbstoffe oder Gemische eines oder mehrerer dieser Komponenten,
jeweils bezogen auf die Gesamtmasse des Verblendmaterials.

Ein besonders bevorzugtes Füllungsmaterial enthält:
I 10 bis 85 Gew.-%, insbesondere 15 bis 60 Gew.-% polymerisierbares Monomer oder polymerisierbare Monomermischung,
II 15 bis 65 Gew.-%, insbesondere 40 bis 65 Gew.-% opaleszenten Füllstoff,
III 5 bis 60 Gew.-%, insbesondere 10 bis 50 Gew.-% weitere übliche Füllstoffe oder Füllstoffgemische und
IV 0,01 bis 5 Gew.-%, insbesondere 0,1 bis 2,0 Gew.-% Polymerisationsinitiatoren, Stabilisatoren, Farbstoffe oder Gemische eines oder mehrerer dieser Komponenten,
jeweils bezogen auf die Gesamtmasse des Füllungsmaterials.

Das Ziel der Erfindung wird durch die geeignete Wahl der Füllstoffkomponenten unter Berücksichtigung der Brechungsindices von Füllstoffen und Monomermischung erreicht. Das erfindungsgemäße Dentalmaterial wies überraschenderweise einstellbare Werte für die Transluzenz und Opaleszenz in einer Weise auf, die aus dem bisherigen Stand der Technik nicht bekannt ist.

Erfindungsgemäß werden für Komponente I vorzugsweise Mischungen aus geeigneten radikalisch polymerisierbaren monofunktionellen und/oder mehrfunktionellen Monomeren, insbesondere di-, tri- und tetrafunktionellen, ganz besonders bevorzugt difunktionellen Vernetzermonomeren, verwendet.

Unter monofunktionellen Monomeren werden Verbindungen mit einer radikalisch polymerisierbaren Gruppe unter mehrfunktionellen Monomeren Verbindungen mit zwei und mehr radikalisch polymerisierbaren Gruppen verstanden. Für die Herstellung von Adhäsiven, Beschichtungsmaterialien und Dentalmaterialien eignen sich vor allem vernetzende di- oder mehrfunktionelle Acrylate oder Methacrylate, wie z.B. Bisphenol-A-di(meth)acrylat, Bis-GMA (das Additionsprodukt von Methacrylsäure und Bisphenol-A-diglycidylether), UDMA (das Additionsprodukt von Hydroxyethylmethacrylat und 2,2,4-Trimethyl-hexamethylendiisocyanat), Di-, Tri- oder Tetraethylenglykoldi(meth)acrylat, Decandioldi(meth)acrylat, Trimethylolpropantri(meth)acrylat und Pentaerithrittetra(meth)acrylat. Es eignen sich ebenfalls die durch Veresterung von (Meth)acrylsäure mit den entsprechenden Diolen zugänglichen Verbindungen Butandioldi(meth)acrylat, 1,10-Decandioldi(meth)acrylat und 1,12-Dodecandiol-di(meth)acrylat, sowie di- und mehrfunktionelle 2-Vinylcyclopropanderivate, die durch Umsetzung von 1-Methoxycarbonyl-2-vinylcyclopropan-1-carbonsäure mit zwei- oder mehrwertigen OH- oder NH₂-Verbindungen als Kopplungskomponente, d.h. z.B. Ethylenglykol, Di- oder Triethylenglykol, Butylenglykol, 1,6-Hexandiol, Glycerin, Pentaerithrit oder Glucose, sowie Hydrochinon, Resorcin, Brenzkatechin oder Pyrogallol, Ethylendiamin, Propylendiamin, Hexamethylendiamin, o-, p- oder m-Phenylendiamin, zugänglich sind.

Weitere mehrfunktionelle radikalisch polymerisierbare Monomere, die sich besonders als Vernetzermonomere eignen, sind Urethane aus 2-(Hydroxymethyl)acrylsäureethylester und Diisocyanate, wie z.B. 2,2,4-Trimethylhexamethylendiisocyanat oder Isophorondiisocyanat, vernetzende Pyrrolidone, wie z.B. 1,6-Bis(3-vinyl-2-pyrrolindonyl)-hexan oder kommerziell zugängliche Bisacrylamide, wie Methylen- oder Ethylenbisacrylamid bzw. Bis(meth)acrylamide, wie z.B. N,N'-Diethyl-1,3-bis(acrylamido)-propan, 1,3-Bis(methacrylamido)-propan, 1,4-Bis(acrylamido)-butan oder N,N'-Bis(acryloyl)-piperazin, die durch Umsetzung aus den entsprechenden Diaminen mit (Meth)acrylsäurechlorid synthetisiert werden können. Diese Verbindungen zeichnen sich außerdem durch eine relativ hohe Hydrolysebeständigkeit aus.

Bevorzugte monofunktionelle radikalisch polymerisierbare Monomere, die sich besonders als Verdünnermonomere eignen, sind hydrolysestabile Mono(meth)acrylate, wie z.B. Mesitylmethacrylat, oder 2-(Alkoxymethyl)acrylsäuren, wie 2-(Ethoxymethyl)acrylsäure, 2-(Hydroxymethyl)acrylsäure, N-mono- oder -disubstituierte Acrylamide, wie z.B. N-Ethylacrylamid, N,N-Dimethylacrylamid, N-(2-Hydroxyethyl)-acrylamid oder N-(2-Hydroxyethyl)-N-methylacrylamid sowie N-mono-substituierte Methacrylamide, wie z.B. N-Ethylmethacrylamid oder N-(2-Hydroxyethyl)-methacrylamid oder auch tert.-Butylmethacrylat, Allylmethacrylat, Isooctylacrylat, 2-(2-Ethoxyethoxy)-Ethylacrylat, Octyldecylacrylat, Laurylmethacrylat, Tridecylmethacrylat, Propylenglykolmonomethacrylat, 2-Ethoxyethylacrylat, tert.-Butylacrylat, Laurylacrylat und Isobornylacrylat.

Zur Reduzierung der Oberflächenenergie ist es bekannt, fluorsubstituierte Monomere zusätzlich in den Mischungen oder allein einzusetzen, da dadurch die Neigung zur Plaquebildung und -anlagerung auf der Zahnoberfläche vermindert werden kann.

Bevorzugte fluorierte monofunktionelle Monomere sind 2,2,2-Trifluorethyl(meth)acrylat, Pentafluormethylmethacrylat, 2-(Pentafluor-butyl)ethyl-(meth)acrylat, 2,2,3,3-Tetrafluorpropyl(meth)acrylat, 3-(Penta-fluor-butyl)-2-hydroxypropyl(meth)acrylat, Perfluorcyclohexylmethylmethacrylat, 3-(Perfluorhexyl)-2-hydroxypropyl(meth)acrylat, 2-(Perfluor-3-methyl-butyl)ethyl-methacrylat, 3-(Perfluor-3-methylbutyl)-2-hydroxypropyl(meth)acrylat, 1H,1 H,5H-Octafluorpentyl(meth)acrylat, 1H,1 H,2H,2H-Pentafluordecylacrylat, 1H,1H-Perfluor-n-decyl(meth)acrylat, 2-(Perfluordecyl)ethyl(meth)acrylat, 2-(Perfluor-9-methyldecyl)ethyl(meth)acrylat, 2-(Perfluor-5-methylhexyl)ethyl(meth)acrylat, 2-(Perfluor-7-methyloctyl)-ethyl(meth)acrylat, 1 H,1 H,7H-Dodecafluorheptyl(meth)acrylat, 1 H,1 H-Perfluoroctyl(meth)acrylat, 1 H,1 H,2H,2H-Perfluoroctyl(meth)acrylat, 1H,1H,9H-Hexadecafluornonyl(meth)acrylat und 1H, 1H, 1H, 11H-Eicosafluorundecyl(meth)acrylat und 1 H,1 H,2H,2H-Pentafluordecylacrylat.

Bevorzugte fluorierte Vernetzermonomere sind fluoriertes Triethylenglycoldimethacrylat (TEGDMA-F), 2,2,3,3-Tetrafluor-1,4-butandioldimethacrylat, 1H,1H,6H,6H-Perfluor-1,6-hexandioldi(meth)acrylat, 1 H, 1 H, 10H, 10H-Perfluordecandioldi(meth)acrylat, 2,2,3,3,4,4,5,5,6,6,7,7-Dodecafluor-1,8octandioldi(meth)acrylat und fluoriertes Bis-GMA {Bis-GMA-F: 2,2-Bis[(4-(2-hydroxy-3-methacryloyloxy)phenyl]hexafluorpropan.

Der Brechungsindex des eingesetzten Monomeren bzw. der Monomermischungen ist <1,45 gemessen bei 25°C. Vorzugsweise beträgt der Brechungsindex 1,380 bis 1,449. Ganz besonders bevorzugt ist ein Brechungsindex von 1,420 bis 1,447.

Diese Monomere als Hauptbestandteil von Dentalmaterialien werden zur Polymerisation mit einem Initiator für die radikalische Polymerisation und vorzugsweise auch mit zusätzlichen Monomeren, Füllstoffen und ggf. weiteren Hilfsstoffen gemischt. Die so erhaltenen Zusammensetzungen können durch radikalische Polymerisation gehärtet werden. Sowohl die härtbaren Zusammensetzungen als auch die gehärteten Produkte sind Gegenstand der Erfindung.

Als Initiatoren für die radikalische Polymerisation eignen sich die bekannten Initiatoren für die Heiß-, Kalt- und Photohärtung. Geeignete Initiatoren werden beispielsweise in der Encyclopedia of Polymer Science and Engineering, Vol. 13, Wiley-Intersci. Pub., New York etc. 1988, S. 754 ff. beschrieben.

Bevorzugte Initiatoren sind Azoverbindungen, wie Azobis(isobutyronitril) (AIBN) oder Azobis(4-cyanovaleriansäure) oder Peroxide, wie Dibenzoylperoxid, Dilaurylperoxid, tert.-Butylperoctoat, tert.-Butylperbenzoat oder Di-(tert.-butyl)peroxid.

Als Initiatoren für die Heißhärtung eignen sich besonders Benzpinakol und 2,2'-Di(C₁-C₈-alkyl)benzpinakole,

Geeignete Photoinitiatoren für den UV- oder sichtbaren Bereich werden von J.P. Fouassier, J.F. Rabek (Hrsg.), Radiation Curing in Polymer Science, London und New York 1993, S. 155 bis S. 237, beschrieben. Bevorzugte Photoinitiatoren sind Benzoinether, Dialkylbenzilketale, Dialkoxyacetophenone, Acylphosphinoxide, Bisacylphosphinoxide, alpha-Diketone, wie 10-Phenanthrenchinon, Diacetyl, Furil, Anisil, 4,4'-Dichlorbenzil und 4,4'-Dialkoxybenzil und Campferchinon.

Zur Herstellung von Dentalmaterialien sind Dibenzoylperoxid, Campfer-chinon und Acylphosphinoxide bevorzugt.

Zur Beschleunigung der Initiierung durch Peroxide oder alpha-Diketone eignen sich besonders Kombinationen mit aromatischen Aminen. Als Beschleuniger sind zudem Redoxsysteme einsetzbar, insbesondere Kombinationen aus Benzoylperoxid, Lauroylperoxid oder Campherchinon mit Aminen, wie N,N-Dimethyl-p-toluidin, N,N-Dihydroxyethyl-p-toluidin, p-Dimethylaminobenzoesäureethylester oder strukturverwandte Amine.

Darüber hinaus sind auch Redoxsysteme geeignet, die neben Peroxid Ascorbinsäure, ein Barbiturat, eine Sulfinsäure oder Mercaptoverbindungen, wie Mercaptobenzothiazol, 2-Mercaptobenzoxazol bzw. 2-Mercaptobenzimidazol als Reduktionsmittel enthalten.

Zur Verbesserung der mechanischen Eigenschaften oder zur Einstellung der Viskosität können verschiedene organische und anorganische Partikel oder Fasern als Füllstoffe in Dentalmaterialien verwendet werden.

Gegenüber dem Stand der Technik werden erfindungsgemäß härtbare Dentatmaterialien zur Verfügung gestellt, aus denen sich gehärtete Dentalmaterialien mit erheblich höherer Opaleszenz herstellen lassen. Durch die Kombination der Füller aus den Komponenten II und III lassen sich Opaleszenz und Transluzenz gezielt einstellen.

Die erfindungsgemäß in Komponente II eingesetzten opaleszenten Füller unterscheiden sich von denen des Standes der Technik dadurch, dass nicht gemahlener, sondern ein sphärischer Füller eingesetzt wird. Die Korngrößenverteilung der erfindungsgemäß eingesetzten Füller ist deutlich enger als die in der US 6,232,367 eingesetzten.

Die erfindungsgemäß eingesetzten opaleszenten Füllstoffe sind möglichst monodisperse, nahezu ideal sphärische Partikel. Diese können über einen Mahlprozess, wie auch im Patent US 6,232,367 beschrieben, nicht erhalten werden. Erfindungsgemäß werden die Partikel daher über den bekannten Sol-Gel-Prozess und zwar in Anlehnung an den Stöber- Prozess [vgI. W. Stöber et al. in J. Colloid and Interface Science 26, 62 (1968) und 30, 568 (1969), US 3,634,588, EP 0 216 278], hergestellt. Die mittlere Partikelgröße liegt bei 230 ± 50 nm. Besonders bevorzugt sind mittlere Partikelgrößen von 230 ± 20 nm.

Die Partikel des opaleszenten Füllstoffes dürfen nicht stark vom Mittelwert streuen. Erfindungsgemäß ist die Standardabweichung der Partikel vom Mittelwert kleiner als 7 %, besonders bevorzugt kleiner als 5%.
Die Partikel werden vor der Verwendung im Dentalmaterial bevorzugt mit üblichen polymerisierbaren Silanen silanisiert. Dies bewirkt einen besseren Verbund zwischen Matrix und Füllstoff nach der Polymerisation und erhöht die mechanischen Eigenschaften des gehärteten Materials. Der Füllstoff lässt sich aber auch durch die Silanisierung besser in die Matrix einarbeiten und verteilen. Überraschenderweise wurde dadurch auch der Opaleszenzeffekt vergrößert.

Der Brechungsindex des erfindungsgemäß eingesetzten opaleszenten Füllstoffs ist zwischen 1,40 und 1,45, bevorzugt zwischen 1,41 und 1,44.
Die Differenzen der Brechungsindices der Komponenten I und II sind ≤0,04, vorzugsweise ≤0,02, besonders bevorzugt ≤0,01.

Erfindungsgemäß können in Komponente III weitere übliche Füllstoffe oder Füllstoffgemische eingesetzt werden. Diese weisen vorzugsweise eine mittlere Korngröße von 0,005 bis 2,0 µm, vorzugsweise 0,01 bis 0,5 nm auf. Weitere bevorzugte Bereiche liegen zwischen 5 und 1000 nm, besonders bevorzugt 40 nm und 500 nm ganz besonders bevorzugt 80 bis 300 nm und höchst bevorzugt 80 bis 120 nm auf. Der Brechungsindex der weiteren Füllstoffe liegt vorzugsweise über dem der Komponenten I und II. Vorzugsweise liegt der Brechungsindex bei 1,45 bis 1,55, bevorzugt bei 1,46 bis 1,52, ganz besonders bevorzugt bei 1,46 bis 1,48.

Füllstoffe der Komponente III zur Herstellung von Dentalmaterialien wie Befestigungszementen, Beschichtungsmaterialien oder Füllungsmaterialen sind amorphe, kugelförmige Materialien auf der Basis von Oxiden, wie SiO₂, ZrO₂, TiO₂ bzw. Mischoxiden aus SiO₂, ZrO2, und/oder TiO₂ mit einer mittleren Primärpartikelgröße von 0,005 bis 1,0 µm insbesondere von 0,01 bis 0,3 µm, nanopartikuläre oder mikrofeine Füllstoffe, wie pyrogene Kieselsäure oder Fällungskieselsäure sowie röntgenopake Füllstoffe, wie z.B. Ytterbiumfluorid, nanopartikuläres Tantal(V)oxid oder Bariumsulfat.

Die erfindungsgemäß einsetzbaren Nanofüller weisen eine mittlere Korngröße von ≤ 40 nm, vorzugsweise 5 bis 25 nm besonders bevorzugt 10 bis 20 nm auf.

Der Anteil des Nanofüllers im Monomer kann 2 bis 10 Gew.-%, vorzugsweise 5 bis 10 Gew.-%, bezogen auf das Monomer betragen.

Vorzugsweise handelt es sich bei den eingesetzten Nanofüllern um kugelförmige, nicht agglomerierte Nanofüller. Beispielsweise kann ein sogenanntes Organosol der Firmen Clariant oder der Hanse Chemie als Füller eingesetzt werden. In diesem Organosol sind die Partikel mit einem polymerisierbaren Silan oberflächenbehandelt und in einem polymerisierbaren Monomer dispergiert.

Als organische Füllstoffe werden sehr häufig auch sog. Kompositfüllstoffe verwendet, die aus einem Monomer oder Monomerengemisch und einem oder mehreren anorganischen Füllstoffen, wie z.B. gemahlenen Gläsern oder verschiedenen SiO₂-Modifikationen oder röntgenopaken Füllstoffen, bestehen. Diese Mischungen werden auf geeignete Weise, vorzugsweise durch Heißhärtung, polymerisiert und anschließend auf eine geeignete Korngröße gemahlen, bevorzugt sind dabei Korngrößen von ca. 0,1 bis 50 µm, besonders bevorzugt 0,2 bis 20 µm.

Der Anteil der organischen Reste im anorganischen opaleszenten Füller liegt vorzugsweise bei ≤5 Gew.-%, besonders bevorzugt bei ≤3 Gew.-%.

Darüber hinaus können die erfindungsgemäßen Zusammensetzungen im Bedarfsfall weitere Hilfsstoffe enthalten, insbesondere Stabilisatoren, UV-Absorber, Farbstoffe, Pigmente und/oder Gleitmittel. Unter Stabilisatoren werden solche verstanden, die eine vorzeitige Polymerisation verhindern und damit vor allem die Lagerstabilität von Monomermischungen und Materialien erhöhen, ohne jedoch die Eigenschaften der ausgehärteten Materialien zu beeinträchtigen. Bevorzugte Stabilisatoren sind Hydrochinonmonomethylether (MEHQ) und 2,6-Di-tert.-butyl-4-methylphenol (BHT).

Die erfindungsgemäßen Zusammensetzungen eignen sich besonders als Dentalmaterialien, insbesondere als Füllungsmateriale, Befestigungszemente oder Beschichtungsmaterialien sowie Materialien für Inlays/Onlays, Zähne oder Verblendmaterialien für Kronen und Brücken. Sie zeichnen sich neben einem geringen Polymerisationsschrumpf und ausgezeichneten mechanischen Eigenschaften vor allem durch ihre hohe Transluzenz und einstellbare Opaleszenz aus.

Nachfolgend wird die Erfindung unter Bezugnahme auf Beispiele näher erläutert, wobei sich die Erfindung nicht auf die aufgeführten Beispiele beschränkt.

### Beispiel 1:

### Herstellung eines Organosol

"UDMA mit 40 Gew.-% SiO₂ (13 nm)" silanisiert

### Silanisierung der Nanopartikel

Ausgehend von 120 g Highlink^{(R)} OG 502-31 (Fa. Clariant), einer kolloidalen Lösung von SiO₂ mit einer mittleren Korngröße von 13 nm (30 Gew.-% SiO₂ in Isopropanol) wurden 5,36 g gamma-Methacryloxypropyltrimethoxysilan (A 174) zugegeben und 2 Minuten bei Raumtemperatur gerührt. Anschließend wurden 1,17 g 0,5 n HCl zugegeben und das Gemisch 6 Stunden bei Raumtemperatur gerührt. Das auf diese Weise erhaltene Sol enthielt 28,4 Gew.-% SiO₂- Partikel. [Das ursprüngliche Sol wurde bei der Oberflächenmodifizierung durch das Wasser und das Silan von 30 Gew.-% auf 28,4 Gew.-% verdünnt. Aus dem silanisierten Organosol wird nur das oberflächenmodifizierte SiO₂ in der unteren Mischung mit dem Monomer umgesetzt, alle anderen flüchtigen Bestandteile werden entfernt (Isopropanol, der bei der Silanisierung abgespaltene Alkohol und das Wasser), so dass die Endzusammensetzung 40 Gew.-% SiO₂ enthält und knapp 60 Gew.-% Urethandimethacrylat (UDMA). Noch enthalten ist ein kleiner Anteil an Silan aus der Oberflächenmodifizierung.]

### Einarbeitung der Partikel zu 40 Gew.-% in UDMA

Zu 126,53 g des oben beschriebenen Organosol wurden 50,13 g UDMA zugegeben und solange gerührt, bis die Mischung homogen war. Nach Zugabe eines Stabilisators (z.B. MEHQ) wurden die flüchtigen Bestandteile bei 40°C am Rotationsverdampfer entfernt, und es wurden 90 g eines transluzenten hochviskosen Öles erhalten, das 40 Gew.-% SiO₂ und 55,7 Gew.-% UDMA enthält. Die Zusammensetzung der Lösung ist:

| Masse (in g) | Anteil (in Gew.-%) | Komponente |
|---|---|---|
| 36,0 | 40,0 | SiO₂ |
| 3,87 | 4,3 | Aufkondensiertes Silan (bei vollständiger Kondensation) |
| 50,13 | 55,7 | UDMA |

### Beispiel 2:

### Herstellung des opaleszenten Füllers aus SiO₂

Es wurde ein Hydrolysegemisch aus 71,4 g Wasser, 376,2 g Ethanol und 9,0 g 25 Vol.-%iger Ammoniaklösung hergestellt. Dieses Hydrolysegemisch wurde auf 40°C erwärmt, und unter intensivem Rühren wurden 26,4 g Tetraethoxysilan zugegeben. Dieses Gemisch wurde 2 Stunden bei 40°C weiter gerührt. Dabei entstand ein Sol mit einer SiO₂-Primärpartikelgröße von ca. 90 bis 115 nm.

Zu diesem Sol wurde über einen Zeitraum von 10 bis 12 Stunden ein Hydrolysegemisch aus 271,4 g Wasser, 1433,8 g Ethanol und 46,8 g 25 Vol.%iger Ammoniaklösung und parallel 216,7 g Tetraethoxysilan zugegeben. Die Größe der hierbei entstandenen Partikel wurde mittels REM periodisch kontrolliert.

Zu den so erhaltenen Partikeln wurde über einen Zeitraum von ca. 4 Stunden bei 40°C eine Mischung aus 0,96 g gamma-Methacryloxypropyltrimethoxysilan in 59,56 ml Ethanol zugetropft. Diese Mischung wurde noch weitere 2 Stunden bei 40°C gerührt. Dabei wurden die Partikel mit dem Silan oberflächenmodifiziert.
Anschließend wurde das Sol am Rotationsverdampfer bei 40°C von den flüchtigen Bestandteilen befreit. Das erhaltene weiße Pulver wurde abschließend 12 Stunden bei einem Druck von 0,1 mbar und einer Temperatur von 110°C getrocknet.
Die Größe der Partikel betrug 215 nm mit einer Standardabweichung von 4,6%.

### Messprinzipien / Messmethoden

Die Transparenz wird mittels eines Chromameter CT-310 von Minolta gemessen. Dabei wird das durchgelassene Licht durch einen definierten Prüfkörper (d = 1 mm) in Wasser zum durchgelassenen Licht in einer reinen Wasserprobe gemessen. Der erhaltene Wert wird in Prozent angegeben. Die Transparenz des natürlichen Schmelzes schwankt zwischen 45 und 80%. (siehe auch US 6,323,367),

Die Teilchengröße der sphärischen Füllstoffpartikel wurde mittels Rasterelektronenmikroskopie bestimmt. Die Schwankungsbreite wurde durch Ausmessen einer statistisch signifikanten Menge ermittelt.

Die Opaleszenz wird mittels des gekoppelten 3-Filtertransmissionssystem CT-310 und 3-Filteremissionssystem CR-300, beide Geräte von Minolta, gemessen. Dabei wurde das angewendete Beurteilungssystem CIELAB ausführlich in der US 6,232,367 (Spalte 6 f.) beschrieben.

### Füllstoffe:

Alle Füllstoffe, die in den nachfolgenden Beispiele verwendet werden, wurden, soweit im Herstellungsprozess noch nicht geschehen, in einem Mischer durch Zugabe von Wasser und einem Silan mit polymerisierbarer Gruppe (gamma-Methacryloxypropyltrimethoxysilan (A-174)) oberflächenbehandelt. Der Silangehalt schwankt, in Abhängigkeit von der BET- Oberfläche des Füllstoffes, zwischen 4 und 10 Gew.-%, bezogen auf die Gesamtmasse des jeweiligen Füllstoffes.

### Beispiel 3

Für die Herstellung und den Vergleich der verschiedenen Dentalmaterialien wurden die in Tabelle 1 aufgeführten Zusammensetzungen verwendet (alle Angaben, soweit nicht anders angegeben, in Gew.-%):

**Tabelle 1:**

| Monomermischung, | 1 | 2 | 3 |
|---|---|---|---|
| V-466 | 60 | 60 | 60 |
| UDMA | 40 | 20 | - |
| 40 Gew.-% SiO₂ in UDMA | - | 20 | 40 |
| Brechungsindex [%] | 1.4466 | 1.4439 | 1.4406 |

Diesen Monomermischungen werden zwischen 0,01 und 5,0 Gew.-% Initiatoren, Stabilisatoren, Beschleuniger oder Farbstoffe, die aus dem Stand der Technik hinreichend bekannt sind, zugesetzt. Diese Zusätze werden durch Rühren bei Raumtemperatur in der Monomermischung gelöst.

Die Monomermischung wird in einem Planetenmischer vorgelegt und die Füllstoffe portionsweise über einen Zeitraum von 1 Stunde zugegeben. Die resultierende Mischung wird anschließend noch eine weitere Stunde geknetet. Danach wird die erhaltene Mischung während 15 Minuten und einem Vakuum von ca. 160 mbar entlüftet.

Für die Messung von Transparenz und Opaleszenz wird eine Mischung aus einer Monomermischung 2 aus der Tabelle 1 und ein sphärischer Füllstoff mit unterschiedlicher Primärteilchengröße (immer im Verhältnis 35 Gew.-% Monomermischung und 65 Gew.-% Füllstoff) hergestellt. Eine Prüfform (Durchmesser = 20 mm, H = 1 mm) wird mit den Mischungen leicht überfüllt und mit 20 bar verpresst. Anschließend wird der Prüfkörper im Lichtofen Spektramat^{(R)} der Firma Ivoclar Vivadent AG für 2 mal 3 min. belichtet, entformt und die Transparenz bzw. Opaleszenz gemessen.

**Tabelle 2:**

| Probe | Teilchengröße [in nm] | Transparenz [in %] | Opaleszenz [in %] |
|---|---|---|---|
| Nr. 1 | 40 * | 46.3 | 2.96 |
| Nr. 2 | 230 ** | 41.0 | 39.0 |
| Nr. 3 | 500 *** | 6.48 | 6.25 |

| | | | |
|---|---|---|---|
| * OX 50, Degussa AG ** erfindungsgemäßer Füllstoff *** Monosphere^{(R)} 500, Degussa AG | | | |

Den oben aufgeführten Ergebnissen kann man entnehmen, dass sich die Opaleszenz nur in einem engen Größenbereich der Füllstoffteilchen einstellt. Auf grund der Übereinstimmung von Brechungsindex von Monomermischung 1 und den 40 nm-Teilchen ist die Transparenz zwar sehr hoch, aber die Opaleszenz ist nur sehr gering. Nur mit den erfindungsgemäßen 230 nm-Teilchen ist eine hohe Transparenz und gleichzeitig eine hohe Opaleszenz erreichbar.

### Beispiel 4

### Herstellung eines hochtransparenten und opaleszenten Komposits

Die Proben wurden gemäß obiger Beschreibung hergestellt. Als Füllstoff wird der erfindungsgemäße Füllstoff mit einer Primärpartikelgröße von ca. 230 nm aus Probe 2 verwendet. Die Zusammensetzung dieser Proben besteht immer zu 35 Gew.-% aus der Monomermischung und zu 65 Gew.-% aus dem Füllstoff.

**Tabelle 3**

| Probe | Monomermischung | Brechungsindex Monomer | Transparenz [in %] | Opaleszenz [in %] |
|---|---|---|---|---|
| Nr. 4 | 1 | 1.4466 | 26.5 | 32.2 |
| Nr. 5 | 2 | 1.4439 | 49.2 | 40.2 |
| Nr. 6 | 3 | 1.4406 | trüb | Trüb |

Durch einen definierten Anteil von Monomer mit nanoskaligen Teilchen im Monomer kann die Transparenz der resultierenden Mischung erhöht werden. Wird jedoch die Grenze noch oben überschritten, kommt es durch Unverträglichkeiten der Mischung zu einer Eintrübung. Der Brechungsindex der Monomermischung muss unter 1,45 liegen, um eine hohe Transparenz und gleichzeitig eine hohe Opaleszenz zu erzielen.
Die Schmelzschicht des natürlichen Zahns besitzt opaleszente Eigenschaften. Zusätzlich ist sie hochtransparent. Je nach Anwendung des opaleszenten Komposits als ästhetisches Füllungsmaterial oder als fließfähiges Material ist die Anforderung nach Opaleszenz unterschiedlich. Da es sich um die oberste Schicht der Restauration handelt, muß manchmal die Transparenz hoch behalten werden und die Opaleszenz wird stufenweise reduziert.

### Beispiel 5

### Komposit mit hoher Transparenz und abgestufter Opaleszenz

Für die Messungen wurden Prüfkörper aus der Monomermischung 2 (35 Gew.-%) und zwei sphärischen Füllstoffen (zusammen 65 Gew.-%) hergestellt:

**Tabelle 4**

| Probe | Opaleszenter Füller (230 nm) | Füllstoff NX 10 sil.**** | Transparenz | Opaleszenz |
|---|---|---|---|---|
| | [in Gew.-%] | [in Gew.-%] | [in %] | [in %] |
| Nr. 7 | 65 | 0 | 63.9 | 39.7 |
| Nr. 8 | 55 | 10 | 57.0 | 37.9 |
| Nr. 9 | 45 | 20 | 52.4 | 34.8 |
| Nr. 10 | 10 | 55 | 51.6 | 22.5 |

| | | | | |
|---|---|---|---|---|
| **** NX 10 sil. = silanisierte sphärische Kieselsäure mit ca. 100 nm Primärteilchengröße der Fa. Degussa AG | | | | |

Durch die Zugabe von Anteilen eines weiteren sphärischen Füllstoffs mit einer Teilchengröße von ca. 100 nm kann die Opaleszenz nach Anforderung reduziert werden, ohne die Transparenz signifikant zu verändern. Die Unterschiede einer Transparenz von 50 % und von 70 % sind vom menschlichen Auge praktisch nicht wahrnehmbar.

### Beispiel 6

### Herstellung eines fließfähigen Komposits

Für die Herstellung des fließfähigen Komposits wurden die Monomermischung 2 der Tabelle 1 und der opaleszente Füllstoff mit einer Primärpartikelgröße von ca. 230 nm verwendet.

**Tabelle 5**

| Probe | Füllstoffgehalt (230 nm) [in Gew.-%1] | Opaleszenz [in %] |
|---|---|---|
| Nr. 11 | 20 | 5.5 |
| Nr. 12 | 40 | 12.0 |
| Nr. 13 | 50 | 19.5 |
| Nr. 14 | 60 | 25.2 |
| Nr. 15 | 65 | 38.6 |

Neben der Möglichkeit der gezielten Einstellung der Opaleszenz gem. Beispiel 4 kann diese auch über den Anteil der Monomermischung beeinflusst werden. Ein hoher Monomeranteil reduziert dabei die Opaleszenz.

## Patentansprüche

1. Härtbares Dentalmaterial mit einstellbarer Transluzenz und hoher Opaleszenz enthaltend,
I. wenigstens ein radikalisch polymerisierbares fluorsubstituiertes Monomer oder eine Monomermischung enthaltend ein radikalisch polymerisierbares fluorsubstituiertes Monomer mit einem Brechungsindex von <1,45 gemessen bei 25°C,
II. wenigstens einen sphärischen, monodispersen, opaleszenten, anorganischen Füllstoff mit einem Brechungsindex von 1,40 bis 1,45 und einer mittleren Partikelgröße bestimmt mittels Rasterelektronenmikroskopie von 230 nm +/- 50 nm,
III. weitere übliche Füllstoffe oder Füllstoffgemische und
IV. Polymerisationsinitiatoren, Stabilisatoren, Farbstoffe oder Gemische eines oder mehrere dieser Substanzen, wobei
die Füller in Komponente III amorphe kugelförmige Materialien auf Basis von Oxiden sind, wobei
die Monomere den Hauptbestandteil der Dentalmaterialien bilden und die Dentalmaterialien einen Initiator für die radikalische Polymerisation enthalten.

2. Dentalmaterial nach Anspruch 1, **dadurch gekennzeichnet, dass** das Monomer oder die Monomermischung einen Brechungsindex von 1,380 bis 1,449 aufweisen.

3. Dentalmaterial nach einem der Ansprüche 1 oder 2, **dadurch gekennzeichnet, dass** die Differenz der Brechungsindices der Komponenten I. und II. ≤0,04 ist.

4. Dentalmaterial nach einem der Ansprüche 1 bis 3, **dadurch gekennzeichnet, dass** der Anteil der organischen Reste im anorganischen opaleszenten Füller ≤5 Gew.-% ist.

5. Dentalmaterial nach einem der Ansprüche 1 bis 4, **dadurch gekennzeichnet, dass** die Füllstoffe oder Füllstoffgemische gemäß Komponente III eine mittlere Korngröße bestimmt mittels Rasterelektronenmikroskopie von 5 nm - 1000 nm aufweisen.

6. Dentalmaterialien nach einem der Ansprüche 1 bis 5, **dadurch gekennzeichnet, dass** es als Füllstoffe III SiO₂, ZrO₂, TiO₂ oder Gemische dieser Stoffe enthält.

7. Dentalmaterial nach einem der Ansprüche 1 bis 6, **dadurch gekennzeichnet, dass** es als Komponente III nanopartikuläre oder mikrofeine Füllstoffe, vorzugsweise pyrogene Kieselsäure, Fällungskieselsäure oder röntgenopake Füllstoffe oder Gemische dieser Substanzen enthält.

8. Dentalmaterial nach einem der Ansprüche 1 bis 7, **dadurch gekennzeichnet, dass** Komponente III als Füllstoff Ytterbiumfluorid, Tantal(V)oxid oder Bariumsulfat oder Gemische dieser Stoffe enthält.

9. Dentalmaterial nach einem der Ansprüche 7 oder 8, **dadurch gekennzeichnet, dass** der Anteil des Nanofüllers mit einer mittleren Korngröße ≤40 nm im Monomer 2 - 10 Gew.-% bezogen auf das Monomer beträgt.

10. Dentalmaterial nach einem der Ansprüche 1 bis 9, **dadurch gekennzeichnet, dass** es in Komponente III organische Füllstoffe enthält oder Kompositfüllstoffe enthält, die ein Monomer oder Monomerengemisch und ein oder mehrere anorganische Füllstoffe enthalten.

11. Dentalmaterial nach Anspruch 10, **dadurch gekennzeichnet, dass** es als anorganische Füllstoffe gemahlenes Glas oder SiO₂-Modifikationen oder röntgenopake Füllstoffe enthält.

12. Dentalmaterial nach Anspruch 11 **dadurch gekennzeichnet, dass** die Füllstoffe eine Korngröße bestimmt mittels Rasterelektronenmikroskopie von 0,1-50 µm aufweisen.

13. Dentalmaterial nach einem der Ansprüche 1 bis 12, **dadurch gekennzeichnet, dass** der Füllstoff oder das Füllstoffgemisch gemäß Komponente III einen Brechungsindex zwischen 1,45 und 1,55 aufweist.

14. Verwendung der härtbaren Dentalmaterialien nach einem der Ansprüche 1 bis 13 zur Herstellung von gehärteten Dentalmaterialien mit einer einstellbaren Transluzenz und hohen Opaleszenz, insbesondere von hoch ästhetischen Füllungsmaterialien.

## Claims

1. Curable dental material having adjustable translucence and high opalescence comprising
I. at least one free radical-polymerizable fluoro-substituted monomer or one monomer mixture comprising a free radicalpolymerizable fluoro-substituted monomer having a refractive index of < 1.45 measured at 25°C,
II. at least one spherical, monodisperse, opalescent, inorganic filler having a refractive index of 1.40 to 1.45 and a mean particle size, determined by means of scanning electron microscopy, of 230 nm ± 50 nm,
III. further customary fillers or filler mixtures and
IV. polymerization initiators, stabilizers, dyes or mixtures of one or more of these substances, where
the fillers in component III are amorphous spherical materials based on oxides, where the monomers form the main constituent of the dental materials and the dental materials contain an initiator for the free-radical polymerization.

2. Dental material according to Claim 1, **characterized in that** the monomer or the monomer mixture has a refractive index of 1.380 to 1.449.

3. Dental material according to one of Claims 1 or 2, **characterized in that** the difference between the refractive indices of the components I. and II. is ≤ 0.04.

4. Dental material according to one of Claims 1 to 3, **characterized in that** the content of the organic radicals in the inorganic opalescent filler is ≤ 5% by weight.

5. Dental material according to one of Claims 1 to 4, **characterized in that** the fillers or filler mixtures in accordance with component III have a mean particle size, determined by means of scanning electron microscopy, of 5 nm - 1000 nm.

6. Dental material according to one of Claims 1 to 5, **characterized in that** the fillers III contained are SiO₂, 2rO₂, TiO₂ or mixtures of these substances.

7. Dental material according to one of Claims 1 to 6, **characterized in that** as component III it contains nanoparticulate or microfine fillers, preferably pyrogenic silica, precipitated silica or X-rayopaque fillers or mixtures of these substances.

8. Dental material according to one of Claims 1 to 7, **characterized in that** component III contains ytterbium fluoride, tantalum(V) oxide or barium sulphate or mixtures of these substances as filler.

9. Dental material according to one of Claims 7 or 8, **characterized in that** the content of the nanofiller having a mean particle sizes 40 nm in the monomer is 2 - 10% by weight, based on the monomer.

10. Dental material according to one of Claims 1 to 9, **characterized in that** in component III it contains organic fillers or contains composite fillers, which contain a monomer or monomer mixture and one or more inorganic fillers.

11. Dental material according to Claim 10, **characterized in that** the inorganic fillers contained are ground glass or SiO₂ modifications or X-ray-opaque fillers.

12. Dental material according to Claim 11, **characterized in that** the fillers have a particle size, determined by means of scanning electron microscopy, of 0.1 - 50 µm.

13. Dental material according to one of Claims 1 to 12, **characterized in that** the filler or the filler mixture in accordance with component III has a refractive index of between 1.45 and 1.55.

14. Use of the curable dental materials according to one of Claims 1 to 13 for the preparation of cured dental materials having an adjustable translucence and high opalescence, in particular of highly aesthetic filling materials.

## Revendications

1. Matériau dentaire durcissable présentant une translucidité réglable et une opalescence élevée, contenant
I. au moins un monomère substitué par fluor, polymérisable par voie radicalaire ou un mélange de monomères contenant un monomère substitué par fluor, polymérisable par voie radicalaire présentant un indice de réfraction mesuré à 25°C < 1,45,
II. au moins une charge sphérique, monodispersée, opalescente, inorganique présentant un indice de réfraction de 1,40 à 1,45 et une grosseur moyenne de particule, déterminée par microscopie électronique à balayage, de 230 nm +/- 50 nm,
III. d'autres charges ou mélanges de charges usuels et
IV. des initiateurs de polymérisation, des stabilisants, des colorants ou des mélanges d'une ou plusieurs de ces substances,
les charges dans le composant III étant des matériaux sphériques amorphes à base d'oxydes,
les monomères formant le constituant principal du matériau dentaire et les matériaux dentaires contenant un initiateur pour la polymérisation par voie radicalaire.

2. Matériau dentaire selon la revendication 1, **caractérisé en ce que** le monomère ou le mélange de monomères présente un indice de réfraction de 1,380 à 1,449.

3. Matériau dentaire selon l'une quelconque des revendications 1 ou 2, **caractérisé en ce que** la différence des indices de réfraction des composants I et II est ≤ 0,04.

4. Matériau dentaire selon l'une quelconque des revendications 1 à 3, **caractérisé en ce que** la proportion de radicaux organiques dans la charge opalescente inorganique est ≤ 5% en poids.

5. Matériau dentaire selon l'une quelconque des revendications 1 à 4, **caractérisé en ce que** les charges ou les mélanges de charges selon le composant III présente une grosseur moyenne de particule, déterminée par microscopie électronique à balayage, de 5 nm1000 nm.

6. Matériaux dentaires selon l'une quelconque des revendications 1 à 5, **caractérisés en ce qu'**ils contiennent comme charges III du SiO₂, du ZrO₂, du TiO₂ ou des mélanges de ces substances.

7. Matériau dentaire selon l'une quelconque des revendications 1 à 6, **caractérisé en ce qu'**il contient, comme composant III, des charges nanoparticulaires ou microfines, de préférence de la silice pyrogène, de la silice précipitée ou des charges opaques aux rayons X ou des mélanges de ces substances.

8. Matériau dentaire selon l'une quelconque des revendications 1 à 7, **caractérisé en ce que** le composant III contient, comme charge, du fluorure d'ytterbium, de l'oxyde de tantale (V) ou du sulfate de baryum ou un mélange de ces substances.

9. Matériau dentaire selon l'une quelconque des revendications 7 ou 8, **caractérisé en ce que** la proportion de la nanocharge présentant une grosseur moyenne de particule ≤ 40 nm dans le monomère représente 2-10% en poids par rapport au monomère.

10. Matériau dentaire selon l'une quelconque des revendications 1 à 9, **caractérisé en ce qu'**il contient, dans le composant III, des charges organiques ou des charges composites, qui contiennent un monomère ou un mélange de monomères et une ou plusieurs charges inorganiques.

11. Matériau dentaire selon la revendication 10, **caractérisé en ce qu'**il contient, comme charges inorganiques, du verre broyé ou des modifications de SiO₂ ou des charges opaques aux rayons X.

12. Matériau dentaire selon la revendication 11, **caractérisé en ce que** les charges présentent une grosseur moyenne de particule, déterminée par microscopie électronique à balayage, de 0,1-50 µm.

13. Matériau dentaire selon l'une quelconque des revendications 1 à 12, **caractérisé en ce que** la charge ou le mélange de charges selon le composant III présente un indice de réfraction entre 1,45 et 1,55.

14. Utilisation des matériaux dentaires durcissables selon l'une quelconque des revendications 1 à 13 pour la préparation de matériaux dentaires durcis présentant une translucidité réglable et une opalescence élevée, en particulier de matériaux d'obturation hautement esthétiques.
